# EUROPEAN PATENT SPECIFICATION

(11) **EP 2 138 048 B1**
(45) Date of publication and mention of the grant of the patent: **18.11.2015**
(21) Application number: 08736726.4
(22) Date of filing: 07.03.2008
(51) Int. Cl.: A23G 3/32, B01J 38/48

(54) **NOVEL CARAMELS WITH A HIGH PREBIOTIC OLIGOSACCHARIDE CONTENT**
NEUE KARAMELLEN MIT HOHEM GEHALT AN PREBIOTISCHEN OLIGOSACCHARIDEN
NOUVEAUX CARAMELS À TENEUR ÉLEVÉE EN OLIGOSACCHARIDES PRÉBIOTIQUES, PROCÉDÉ DE PRÉPARATION ET UTILISATION

(30) Priority: 08.03.2007 ES 200700675
(43) Date of publication of application: 30.12.2009
(73) Proprietor: Universidad de Sevilla, 41013 Sevilla (ES); Consejo Superior De Investigaciones Científicas, 28006 Madrid (ES); Universidad de Granada, 18071 Granada (ES)
(72) Inventor: RUBIO CASTILLO, Enrique, Miguel, E-41012 Sevilla (ES); GÓMEZ GARCÍA, Marta, E-41012 Sevilla (ES); ORTIZ MELLET, Carmen, E-41012 Sevilla (ES); GARCÍA FERNÁNDEZ, José, Manuel, E-41092 Sevilla (ES); ZARZUELO ZURITA, Antonio, E-18071 Granada (ES); GÁLVEZ PERALTA, Julio, Juan, E-18071 Granada (ES); DUVAL, Raphael, F-76190 Allouville Bellefosse (FR)
(74) Representative: Temino Ceniceros, Ignacio
(86) International application number: PCT/ES2008/000129
(87) International publication number: WO 2008/107506

(56) References cited:
- EP-A1- 1 834 957
- WO-A1-94/12049
- WO-A2-03/038014
- US-A- 5 925 190
- DATABASE WPI Week 199023 Thomson Scientific, London, GB; AN 1990-175283 XP002728131, & JP H02 115193 A (MITSUBISHI KASEI CORP) 27 April 1990 (1990-04-27)
- MANLEY-HARRIS M ET AL: "Dihexulose dianhydrides", ADVANCES IN CARBOHYDRATE CHEMISTRY AND BIOCHEMISTRY, ACADEMIC PRESS, vol. 52, 1 January 1997 (1997-01-01), pages 207-266, XP009179468, ISSN: 0065-2318
- RATSIMBA V ET AL: "Qualitative and quantitative evaluation of mono- and disaccharides in d-fructose, d-glucose and sucrose caramels by gas-liquid chromatography-mass spectrometry", JOURNAL OF CHROMATOGRAPHY, ELSEVIER SCIENCE PUBLISHERS B.V, NL, vol. 844, no. 1-2, 4 June 1999 (1999-06-04), pages 283-293, XP027231994, ISSN: 0021-9673 [retrieved on 1999-06-04]
- DEFAYE J ET AL: "The oligosaccharide components of caramel", ZUCKERINDUSTRIE - SUGAR INDUSTRY - INDUSTRIE SUCRIRE, BARTENS, BERLIN, DE, vol. 120, no. 8, 1 January 1995 (1995-01-01), pages 700-704, XP009179479, ISSN: 0344-8657

## Description

### OBJECT OF THE INVENTION

The present invention relates to a new method of producing caramels with a high content in oligosaccharides with prebiotic activity and the caramel products produced thereby. The present invention also relates to the use of these caramels as ingredients or additives in the elaboration of food products for animal feeding or of specific food products for humans. More precisely, the present invention relates to the transformation of food-grade sugars in caramels enriched in oligosaccharides with prebiotic activity by using solid acid catalysts, such as zeolites, clays or ion-exchange resins in its acid form, under heterogeneous conditions, or by using soluble acid polymers of high molecular weight as catalysts. An important advantage of the method is the possibility of recycling the catalyst, being compatible with both discontinuous and continuous production processes. According to this invention, the starting food-grade sugar can be D-fructose, sucrose or any oligo- or polysaccharide containing fructose as constituent, including the glycosylfructoses such as palatinose or leucrose, fructooligosaccharides such as 1-kestose or nystose, fructans and inulin. These starting sugars can be used alone or in combination in different proportions, as well as in combination with other food-grade sugars, including glucose, galactose, maltose, lactose or raffinose. The resulting products of the activation of these sugars with the indicated catalysts display a high proportion of fructose-containing oligosaccharides and exhibit prebiotic properties, favoring the development of a beneficial intestinal flora, in particular Bifidobacteria and Lactobacillus, and exerting a repairing effect in the damaged colon.

### STATE OF THE TECHNIQUE

The oligosaccharides that contain D-fructose in their structure named generically fructooligosaccharides, have demonstrated to have beneficial nutritional properties when incorporated in the animal as well as in the human diet. These oligosaccharides modify the intestinal flora favoring, particularly, an increase in the proportion of bacteria of the Bifidus genus in the gut. Consequently, the caramels that contain an elevated proportion of this oligosaccharides present important nutritional advantages.

The caramels are products arising form the heat treatment of sugars, such as sucrose, fructose, glucose or others. This heat treatment can take place on the dry sugar or in the presence of water, in the absence or in the presence of acid or basic additives, salts or nitrogen-containing compounds. Its composition has been studied previously and it consists, basically, in a volatile fraction in which the major component is 2-hidroxyimethylfurfural (HMF) and in a nonvolatile fraction constituted by a variable proportion of the starting sugar or of its monosaccharide components and by oligosaccharides formed from them during the caramelization process. In particular, for the case of industrial caramels prepared from sucrose in the presence of a food-grade acid, the major components of this oligosaccharidic fraction, which can reach up to 20% of the total mass, have the difructose dianhydride structure. Up to 13 different isomers with this general structure, resulting from the dimerization of D-fructose with formation of two reciprocal glycosidic linkages, have been identified in caramels. Higher oligomers, resulting of the addition of D-fructose or D-glucose units, arising from hydrolysis of sucrose during caramelization, on a central nucleus of difructose dianhydride, as well as reversion glucooligosaccharides, are also present in caramel. Both difructose dianhydrides as well as their glycosylated derivatives have shown to have prebiotic properties.

The preparation of caramels enriched in difructose dianhydrides and fructooligosaccharides thereof presents the difficulty associated to the reversible character of the dimerization reaction of fructose and the glycosylation reactions. Moreover, these reactions compete with nonspecific dehydration reactions.

In the documents WO 87/07275, 837 EP 0 252 A1, 788 FR 2 680 789 A1 and FR 2 680 A1, Defaye and coworkers have described the use of anhydrous hydrogen fluoride or acid reagents derived from hydrogen fluoride, such as poly(hydrogen fluoride) piridinium, to favor the formation of difructose dianhydrides and their glycosylated derivatives from fructose, sucrose, fructooligosaccharides or inulin. Although high conversions into oligosaccharides are reached, the use of hydrogen fluoride presents technical difficulties associated to its toxicity, its corrosive character and to the elimination of fluorine traces in the final product.

In the document U.S. 5 454 874, Richards has described the preparation of caramels with a high content in fructooligosaccharides by a procedure that consists of intimately milling sucrose and a food-grade acid, preferably citric or tartaric acid, both components finely divided, and subjecting the mixture to heat treatment (130-160 °C), The product thus obtained contains between 20 and 50% of fructooligosaccharides, including difructose dianhydrides and their glicosilated derivatives, with a degree of polymerization (DP) ranging from 2 to 20.

In the document WO 96/39444, the same author has extended the previous method to the preparation of caramels enriched in difructose dianhydrides and higher oligomers from the polysaccharide inulin by pyrolysis at 150-205 °C. In this case, the composition of the resulting product has been studied in detail. In particular, it was established that the relative proportions of the different difructose dianhydride isomers present in the final product do not correspond with a thermodynamic distribution, differing substantially from the product that is obtained by activation with hydrogen fluoride.

An inherent problem of the above commented methods, in which caramelization takes place under homogenous conditions, is that the acid catalyst used to promote the formation of the fructooligosaccharides is present in the final product. In the case of the use of hydrogen fluoride, its elimination represents an additional cost and entails a considerable risk. The food-grade acids, on the other hand, are much weaker acids and they lead to conversions in fructooligosaccharides that, generally, are lower than 50%. In addition, the fact that the catalyst remains in the final product limits the proportion in which it can be used and is going to affect significantly the organoleptic properties of resulting caramels.

An additional problem of the previous procedures is that the used of weak acids as caramelization promoters results in kinetic distributions of difructose dianhydrides that, since they are not in thermodynamic equilibrium, can evolve with time altering the composition of the product. This is aggravated by the fact that the isomerization and nonspecific dehydration reactions are also catalyzed by the acid used as caramelization promoter. In general, in a distribution of difructose dianhydrides close to the thermodynamic equilibrium the major isomer contains one unit of fructose in the pyranose form, whereas in kinetic distributions the major compound contains the two fructose units in the furanose form.

Therefore, there is a need of methods of preparing caramels with a high content of prebiotic oligosaccharides derived from difructose dianhydrides that allow withdrawing the acid catalyst at the end of the process, leading preferentially to well-defined distributions, next to the thermodynamic equilibrium, of the final components.

According to the present invention, we have discovered that the use of solid acid catalysts, such as zeolites, bentonite or ion-exchange resins in its acid form, is able to promote the formation of caramels with a high content in difructose dianhydrides and glycosylated difructose dianhydrides under heterogeneous conditions, starting from fructose, food-grade sugars that contain fructose such as sucrose, glycosylfructoses, fructooligosaccharides, fructans or inulin, or from mixtures of these, or even from mixtures that contain other food-grade sugars like, for example, glucose, galactose, lactose, maltose, or raffinose. The transformation takes place at elevated concentration of the starting sugar or mixture of sugars, preferably in the range 60-95% (weight/volume) in water and with an effective stirring, at temperatures that vary between 60-110 °C, preferably between 70-90 °C, and reaction times that depend on the catalyst, going from 5 minutes to one week, preferably between 15 minutes and 3 hours when the starting sugar is fructose and between 3 and 48 hours when the starting material contains a different sugar. The resulting product can be easily separated from the catalyst by filtration and contains a high proportion of difructose dianhydrides and of glycosylated difructose dianhydrides. The proportion of difructose dianhydrides and of glycosylated difructose dianhydrides can be modulated by adjusting the reaction conditions, varying between 40-85% and being preferably in the range 50-80%. The distribution of different difructose dianhydride isomers in the final caramel is close to that expected for a thermodynamic distribution and, after the separation of the catalyst, it does not experience significant variations upon storage for a 12 months' period.

According to the present invention, caramelization can also take place under homogenous conditions using a soluble acid polymer of high molecular weight as catalyst, obtaining also in this case a product with an elevated content in prebiotic oligosaccharides, preferably between 50-80%. The separation of the catalyst of the final product is carried out, in this case, by the use of membranes that allows separation of the high molecular weight polymers from the prebiotic oligosaccharides.

An additional advantage of the methodology developed in this invention is the possibility of regenerating and recycling the acid catalyst after separation and its compatibility with discontinuous or continuous processes.

The caramels obtained according to the present invention exhibit prebiotic properties and they are usable as ingredients or additives in the elaboration of food for animal feeding or in the elaboration of specific food products intended for humans. Thus, the products obtained in agreement with the present invention favor the development of a beneficial intestinal flora. In particular, they increase the proportion of Bifidobateria and Lactobacillus in models animals. In addition, they show a repairing effect on the damaged colon in a model animal that corresponds with diseases such as Crohn disease in humans. Consequently, the caramels prepared according to this invention can be considered as nutraceuticals useful for the treatment of this pathology and other related pathologies in humans and in animals.

### DESCRIPTION OF THE FIGURES

Figure 1. Structures of the difructose dianhydrides (DFAs) present in sucrose caramels (excepting in compound **8**, the two monosaccharide subunits derive from D-fructose; Fru = D-fructose; Glc = D-glucose; *f*= furanose; *p* = pyranose).
Figure 2. Relative proportions of the different DFA isomers obtained by caramelization of D-fructose (90% w/v in water) with Degussa FAU 110 zeolite (32%) at 90 °C during 3 h.
Figure 3. Relative proportions of the different DFA isomers obtained by caramelization of D-fructose (90% w/v in water) with Lewatit® S2328 ion-exchange resin (20%) at 90 °C during 2 h.

### BRIEF DESCRIPTION OF THE INVENTION

A first object of the present invention is the production of caramels with a high content in prebiotic oligosaccharides from food-grade sugars that contain fructose in their composition, from mixtures of several of these sugars or from mixtures of them with other sugars, by means of procedures that allow the separation of the used acid catalyst at the end of the process in a simple way, typically by filtration, centrifugation or dialysis.

A second object of the present invention is a procedure that allows maximizing the content of prebiotic oligosaccharides of the difructose dianhydride and glycosylated difructose dianhydride type in caramels, favoring preferably isomeric distributions of difructose dianhydrides close to the thermodynamic equilibrium.

According with these objects of the invention and with other objects that are mentioned hereinafter, the present invention provides a procedure for the preparation of caramels with a high content in prebiotic oligosaccharides that includes:
(a) A food-grade sugar as the starting material that is selected, preferably between D-fructose, sucrose or any oligo or polysaccharide that contains fructose as a component, including the glycosylfructoses such as palatinose or leucrose, the fructooligosaccharides like 1-kestose or nystose, fructans and inulin. These starting sugars can be used alone or combined in different proportions, as well as in combination with other food-grade sugars, including glucose, galactose, maltose, lactose or raffinose.
(b) The use of solid acid catalysts, such as zeolites, bentonite or ion-exchange resins in its acid form, under heterogeneous reaction conditions, or soluble acid polymers of high molecular weight under homogeneous reaction conditions.

Preferably, in agreement with the invention, the caramelization is carried out in the presence of water, with total sugar concentrations ranging between 60-95% (weight/volume) in water and with an effective constant stirring, at temperatures ranging between 60-110 °C, preferably between 70-90 °C, and reaction times that can vary from 5 minutes to one week, preferably between 15 minutes and 3 hours when the starting sugar is fructose and between 3 and 48 h when the starting material includes a different sugar.

The present invention also provides new caramels with a high content in difructose dianhydrides and glycosylated difructose dianhydrides, between 40-85%, preferably between 50-80%, with a composition in difructose dianhydride isomers close to that corresponding to a thermodynamic distribution and free of the acid catalyst used as caramelization promoter, as well as the use of these caramels as prebiotics that, among other favorable effects, favor the development of a beneficial intestinal flora, such as Bifidobacteria or Lactobacillus, and that shows a repairing effect on injuries in the colon.

### DETAILED DESCRIPTION OF THE INVENTION

In agreement with the present invention, we have found it possible to prepare caramels with a high content in difructose dianhydrides and glycosylated difructose dianhydrides from food-grade sugars, using solid catalysts, such as zeolites, bentonite or ion-exchange resins in its acid form, or soluble acid polymers of high molecular weight, as caramelization promoters. These oligosaccharides present prebiotic properties, exerting a repairing effect on injuries of the colon and modifying the intestinal flora, increasing the proportion of beneficial bacteria like Bifidobacteria or Lactobacillus in the gut in animals (poultry, pigs, rabbits) and in humans. The caramels with an elevated content in these oligosaccharides show, consequently, important nutritional advantages in comparison with conventional caramels.

The starting sugar can be D-fructose, sucrose or any oligo or polysaccharide that contains fructose as component, including the glycosylfructoses such as palatinose or leucrose, fructooligosaccharides like 1-kestose or nystose, fructans and inulin. These starting sugars can be used alone or combined in different proportions, as well as in combination with other food-grade sugars, including glucose, galactose, maltose, lactose or raffinose. The caramel is prepared using an elevated total sugar concentration in water, between 60-95% and, preferably, between 70-90% (weight/volume), in the presence of a proportion of the catalyst that can vary between 5-35% in weight, referred to the total sugar, preferably between 5-20%, and at temperatures between 60-110 °C, preferably between 80-90 °C.

In the case that the starting sugar is D-fructose, the addition of water gives rise to dissolutions in all the range of concentrations of the invention. In this case, the preferred caramelization times oscillate between 5 minutes and 3 hours. In the case of other sugars like sucrose or inulin, or when using sugar mixtures, suspensions can be initially obtained that, during the process of caramelization in the presence of the catalyst, lead finally to dissolutions. The preferred caramelization times in these cases range between 3 hours and 48 hours. In any case, the final product once separated from the catalyst is a homogenous caramel of amber to dark mahogany color.

In the case of solid catalysts like the zeolites, bentonite or ion-exchange resins, the reaction takes place under heterogeneous conditions In the case of soluble acid polymer catalysts of high molecular weight, the reaction takes place under homogenous conditions in those cases in which the mixture of starting sugar and water gives rise to an initial dissolution, In the cases when a suspension is obtained, the reaction proceeds initially under heterogeneous conditions and evolves to a homogenous dissolution in the course of the caramelization. In all cases, the reaction takes place preferably under vigorous, effective and constant stirring, for example magnetic or mechanical, during the period of heating.

In agreement with the present invention, when the catalyst used for the caramelization is a zeolite in its acid form, it can belong to any of the commercially available families of zeolites, preferably to the families of Faujasite (FAU) or the beta-zeolites (BEA). The modulus of the used zeolite (Si/Al proportion) can vary between 5 and 150, and preferably is comprised between 25 and 120. As examples, the FAU 15, FAU 25/5, FAU 25, FAU 56 or FAU 110 zeolites commercialized by Degussa and the CBV500 and BEA CP814B-50 zeolites commercialized by Zeolyst can be mentioned. In the case of zeolites commercialized in neutral form, they are previously transformed to their acid form prior to its use as caramelization catalysts. For this purpose, a procedure consisting in the exchange of the metal cation present in the commercial neutral form by ammonium cation (NH₄⁺), followed by heating at temperatures between 100-450 °C, which causes the elimination of ammonia (NH₃), affords the zeolite in its acid form (H⁺).

According to another procedure of the invention, the catalyst used during the caramelization can be a commercial bentonite in its acid form. In the case that the commercial catalyst is in the neutral form, it can be conditioned to its acid form following, for example, the procedure above indicated for the case of the zeolites.

According to an advantageous procedure of the present invention, caramelization can be effected using a commercial ion-exchange resin in its acid form, as for example the resins of styrenic or metacrylic matrices carrying sulfonic acid or carboxylic acid groups. As an example, the commercial resins Lewatit® S2328, K1131, K1469 and K2641, Amberlite® IRC50 or IR120 or Dowex® 50WX2 can be mentioned. The resin can be used intact or milled, modifying in this way the particle size. The resin can be used either wet or dry.

In agreement with the invention, when the catalyst used for the preparation of the caramels with high content in prebiotic oligosaccharides is a zeolite, a bentonite or an ion-exchage resin, it is separated of the final product by filtration, eventually after centrifugation. If the caramelization takes place in a continuous process, the catalyst is packed in a column provided with a filter with porosity adapted to the particle size. In the case of preparations conducted by discontinuous batches, the centrifugation/filtration of the catalyst is carried out at the end of the heating process.

According to another procedure of the invention, the caramelization can be also effected using a soluble acid polymer of high molecular weight as catalyst, such as the polymers of poly(*p*-toluenesulfonate) type commercialized by Sigma of molecular weight 7-10⁴ and 10⁶ Dalton. In the case of polymers commercialized in its neutral form, they are first conditioned to their acid form. For this purpose, a procedure consisting in the treatment of an aqueous solution of the polymer with an excess of ion-exchage resin in its acid form, for example the resin Amberlite® IR120, can be followed. Once finished the caramelization process, the polymer is separated of the final product by physical methods. For this purpose, a procedure consisting in the use of a dialysis membrane of porosity adapted to the molecular weight of the catalyst, allowing the passage of the formed prebiotic oligosaccharides, can be followed.

The proportion of catalyst referred to the total initial sugar weight can vary, being preferably in the range 5-35%. Although the use of elevated proportions of catalyst does not represent technical problems, since the catalyst is separated of the final product and can be recycled, it is preferred to adapt the proportion of catalyst to the minimum so that conversions in prebiotic oligosaccharides of the difructose dianhydride and glycosylated difructose dianhydride type higher than 50%, in reaction times shorter than 3 hours at caramelization temperatures of 70-90 °C, are obtained. Generally, the proportion of catalyst ranges between 25-35% in the case of the zeolites or bentonite, between 5-20% in the case of intact ion-exchange resins and between 5-10% for milled ion-exchange resins with particle size <80 µm and soluble acid polymers.

In agreement with the above considerations, the procedure to prepare a caramel with a high content in prebiotic oligosaccharides of the difructose dianhydride and glycosylated difructose dianhydride type according to the present invention consists, essentially, in the heating of a dissolution or suspension of the starting food-grade sugars at high concentration in water in the presence of a solid acid catalyst or a soluble acid polymer, with constant and effective agitation and at temperatures ranging between 60-110 °C, followed by the separation of the catalyst by physical methods.

A preferred procedure to prepare caramels enriched in prebiotic oligosaccharides in agreement with the present invention consists in the heating of a 70-90% (weight/volume) solution of fructose in water at 70-90 °C in the presence of an ion-exchage resin with sulfonic groups in its acid form, using a proportion of catalyst of 5-20% by weight referred to the starting sugar, for a period of 0.5-3 hours, followed by separation of the resin by centrifugation/filtration.

Another preferred procedure to prepare caramels enriched in prebiotic oligosaccharides in agreement with the invention consists in the heating of a solution of fructose and lactose, in relative proportions by weight that can vary from1:5 to 5:1, in a total concentration of 85-95% (weight/volume) in water, at 80-90 °C, in the presence of an ion-exchage resin with sulfonic groups in their acid form, using a proportion of catalyst of 10-20% by weight referred to the starting total sugar, for a period of 3-48 hours, followed by separation of the resin by centrifugation/filtration.

The composition of the resulting final caramel can be determined by gel filtration chromatography and gas chromatography, using additionally structural determination techniques such as mass spectrometry and proton and carbon-13 nuclear magnetic resonance. The degree of polymerization (DP) of the formed prebiotic oligosaccharides ranges from 2 to approximately 25, being generally 2-12 when the starting sugar is fructose and increasing generally to 2-25 when the starting material contains other sugars. The oligosaccharides formed display a broad variety of glycosidic linkages.

The caramels prepared according to the present invention contain proportions of starting sugars or their monosaccharide components that vary between 10-60% and of prebiotic oligosaccharides of the difructose dianhydride and glycosylated difructose dianhydrides type between 40-85%. When the initial sugar contains a monosaccharide different from fructose, the resulting caramel can contain in addition variable amounts of reversion reducing oligosaccharides resulting from self-glycosylation reactions of the said monosaccharide. For example, in the case of caramels obtained from sucrose the presence of glucobioses and higher glucooligosaccharides in proportions generally lower than 10% is detected.

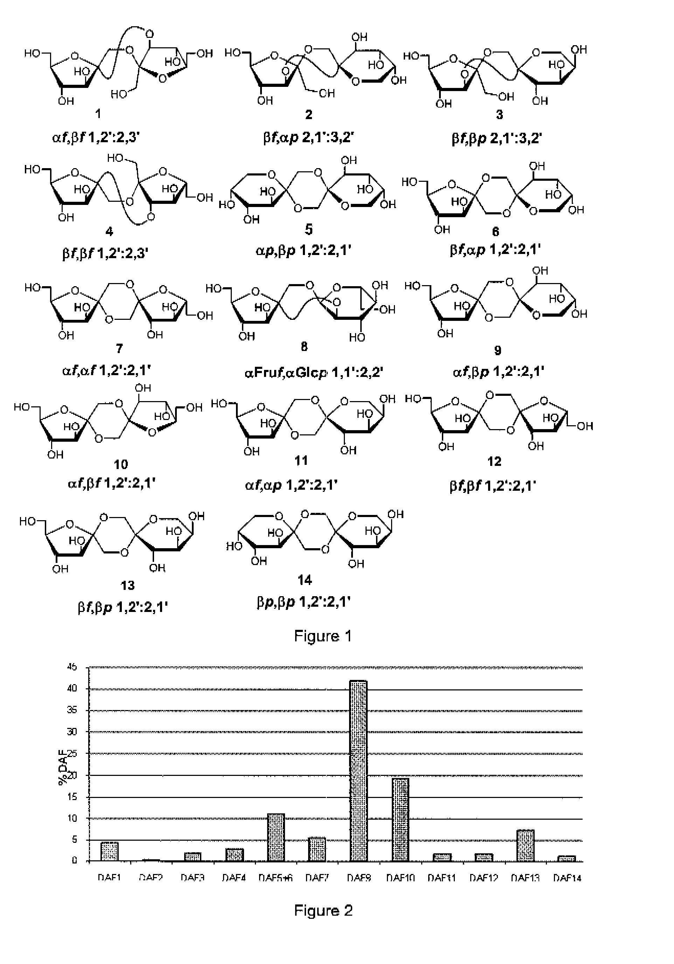

In the caramels prepared according to the present invention, the disaccharide fraction consists mainly of difructose dianhydrides, whereas the higher oligosaccharides have structure of glycosylated difructose dianhydrides, essentially. The isomeric distribution of the different difructose dianhydrides in the disaccharide fraction can be determined by gas chromatography. The protocol described by Ratsimba et al. in the document J. Chromatogr. A. 1999, 844, 283-293 can be followed. The chromatograms obtained from samples of caramels of the invention indicate the presence of 13 isomeric difructose dianhydrides. In the particular case of caramels obtained from sucrose it is identified additionally a mixed dianhydride which contains a subunit of fructose and another of glucose in this fraction. The structures of these dianhydrides correspond with the 13 and 14 structures identified previously in industrial or homemade caramels obtained by heat treatment of D-fructose or sucrose, respectively, in the presence of a food-grade; acid, shown in Figure 1, namely:
- α-D-fructofuranose β-D-fructofuranose 1.2':2,3'-dianhydride (compound n° 1).
- β-D-fructofuranose α-D-fructopyranose 1.2': 2,3'-dianhydride (compound n° 2).
- β-D-fructofuranose β-D-fructopyranose 1.2': 2,3'-dianhydride (compound n° 3).
- Di-β-D-fructofuranose 1.2': 2,3'-dianhydride (compound n°4).
- α-D-fructopyranose β-D-fructopyranose 1.2': 2,1'-dianhydride (compound n° 5).
- β-D-fructofuranose α-D-fructopyranose 1.2': 2,1'-dianhydride (compound n° 6).
- Di-α-D-fructofuranose 1.2': 2,1'-dianhydride (compound n° 7).
- α-D-fructofuranose α-D-glucopyranose 1.1': 2,2'-dianhydride (compound n° 8).
- α-D-fructofuranose β-D-fructopyranose 1.2': 2,1'-dianhydride (compound n° 9).
- α-D-fructofuranose β-D-fructofuranose 1.2': 2,1'-dianhydride (compound n° 10).
- α-D-fructofuranose α-D-fructopyranose 1.2': 2,1'-dianhydride (compound n° 11).
- Di-β-D-fructofuranose 1.2': 2,1'-dianhydride (compound n° 12).
- β-D-fructofuranose β-D-fructopyranose 1.2': 2,1'-dianhydride (compound n° 13).
- Di-β-D-fructopyranose 1.2': 2,1'-dianhydride (compound n° 14).

An important characteristic of the invention is that the relative proportions of the different difructose dianhydride isomers in the resulting caramels correspond, preferably, to distributions close to the thermodynamic equilibrium. Thus, in contrast to that observed in caramels obtained by procedures that use food-grade acids as catalysts, in which the major isomer is always a difructofuranose isomer, preferably the compounds n° 1, 4 or 10, the major isomer in the caramels obtained according to the present invention is compound n° 9, in which one of the two subunits of fructose is in the pyranose form, which is the thermodynamically more stable isomer.

Another important characteristic of the invention is that the prebiotic oligosaccharides with structure of difructose dianhydrides and glycosylated difructose dianhydrides that are the major components of the caramels that are the object of the invention are not toxic and they are not hydrolyzed or they are only partially hydrolyzed during the digestion. In the last case, the products resulting of hydrolysis are food-grade sugars and, consequently, devoid of toxicity. The caramels with elevated content in difructose dianhydrides and glycosylated difructose dianhydrides of the present invention exhibit, therefore, a reduced caloric power in comparison with other caramels of different composition.

The caramels prepared according to the present invention present important nutritional advantages, derived from their elevated content in prebiotic oligosaccharides, in particular of difructose dianhydrides and glycosylated difructose dianhydrides, and from the isomeric distribution close to the thermodynamic equilibrium of the difructose dianhydrides, in comparison with caramels of different composition previously prepared. In tests made on Wistar rats to which an injury in the colon has been induced to generate a model analogous to the Crohn disease in humans, the caramels of the invention have demonstrated to have an important repairing effect, at the same time that they favor the development of a beneficial intestinal flora of Bifidus and Lactobacillus type in the colon. The results indicate that caramels of the present invention exhibit these beneficial effects in greater intensity than some difructose dianhydrides in pure form, like for examples compounds n° 1 and 10, for which the prebiotic properties are well established.

The caramels prepared in agreement with the present invention have numerous applications and can, in a general manner, be used as a substitute of any other caramel. The obtained caramel can be mixed with additional sugars, vitamins, aromas, colorants, with other prebiotics, probiotics or any other substance necessary for the elaboration of a defined food product. The obtained caramel can also be decolorized, for example by the treatment of a water solution with charcoal or with a commercial resin approved for color adsorption in the food industry, as for example the resin Lewatit® S6823 A. This process does no affect the composition in difructose dianhydrides and glycosylated difructose dianhydrides or the relative proportion of the isomeric difructose dianhydrides.

The caramels with elevated content of difructose dianhydrides and glycosylated difructose dianhydrides of the invention have beneficial properties, notably for the treatment and prevention of pathologies of the intestinal tract in animals and in humans. Therefore, they can be also used in the preparation of specific nutraceuticals useful for the prevention and treatment of these pathologies. In a general way, the caramels of the invention can be used as a substitute of other prebiotics in the elaboration of products intended for food applications as well as to improve health and well-being in animals and humans. The final proportion of prebiotic caramel of the invention in a product able to produce a prebiotic effect destined to anyone of these aims can vary in an broad range, being preferably comprise between 1 and 30%.

The following examples are presented to illustrate the invention, and should not be construed as a limitation thereto.

### Example 1:

To a 90% (weight/volume) dissolution of fructose (135 g) in water (15 mL) commercial dry zeolite 110 Degussa FAU (43.2 g; 32% relative to the initial fructose) was added. The heterogeneous mixture was heated at 90 °C in a closed vessel with constant magnetic stirring during 3 hours, after which it was let cool down to room temperature and the catalyst was separated by filtration. The product obtained in this way is an amber-colored caramel.

The analysis of this caramel by gel filtration chromatography using Sephadex G10 as the stationary phase, and by gas chromatography using phenyl β-D-glucopyranoside as internal standard, following the protocol described in the document J. Chromatogr. A. 1999, 844, 283-293, indicated the presence of fructose (35%), difructose dianhydrides (45%) and higher fructooligosaccharides of DP 3-12 (18%). The rest (2%) is constituted essentially by 2-hydroxymethylfurfural (HMF) and melanoidines. The relative proportions of the different isomeric difructose dianhydrides, determined from the corresponding gas chromatogram, are presented in Figure 2.

The mild acid hydrolysis of an aliquot of the obtained caramel or the fraction containing oligosaccharides of DP 3-12 lead exclusively to fructose and difructose dianhydrides, which indicates that these oligosaccharides have a structure of fructosylated difructose dianhydrides. The isomeric distribution profile of the difructose dianhydrides arising from hydrolysis is practically identical to that in the difructose dianhydride fraction in the initial caramel and shown in Figure 2.

### Example 2:

The procedure of example 1 was repeated exactly, using dry acid bentonite as catalyst instead of the zeolite. The product is a mahogany-colored caramel that contains fructose (31%), difructose dianhydrides (46%) and higher oligosaccharides of DP 3-10 (21%). The rest (2%) is constituted essentially by 2-hydroxymethylfurfural (HMF) and melanoidines. The isomer distribution profile of difructose dianhydrides is practically identical to that in example 1.

### Example 3:

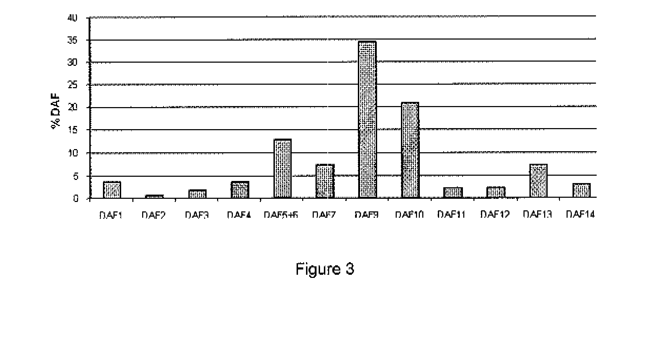

The procedure of example 1 was repeated using the dry commercial ion-exchange resin Lewatit® S2328 as catalyst (27 g, 20% by weight relative to the initial fructose) instead of the zeolite and heating at 90 °C during 2 hours. The product is a mahogany-colored caramel that contains fructose (8%), difructose dianhydrides (11%) and higher fructooligosaccharides of DP 3-25 (78%). The rest (3%) is constituted essentially by 2-hydroxymethylfurfural (HMF) and melanoidines. The isomer distribution profile is very similar to that in examples 1 and 2 and is shown in Figure 3.

Practically identical results were obtained following this procedure but replacing the resin Lewatit® S2328 by another ion-exchange resin with sulfonic groups in its acid form selected among the commercial ion-exchange resins Lewatit® K1131, K1469 or K2641, Amberlite® JR120 or Dowex® 50WX2.

### Example 4:

The procedure of example 3 was repeated exactly, but the resin was previously milled to a size of less than 80 µm, it was used in a 6% proportion by weight relative to the initial fructose and the heating took place at 70 °C. The product is a mahogany-colored caramel that contains fructose (12%), difructose dianhydrides (41%) and higher fructooligosaccharides of DP 3-25 (44%). The rest (3%) is constituted essentially by 2-hydroxymethylfurfural (HMF) and melanoidines. The profile of isomer distribution is practically identical to that shown in Figure 2.

### Example 5:

The procedure of example 4 was repeated exactly, but the heating took place at 90 °C during 50 minutes. The product is a dark mahogany-colored caramel with a composition identical to that in example 3.

### Example 6:

The procedure of example 3 was repeated exactly, but the acid resin was used in a proportion of 10% by weight relative to the initial fructose and heating was effected during 1.5 hours. The product is a dark mahogany-colored caramel that contains fructose (14%), difructose dianhydrides (26%) and higher fructooligosaccharides of DP 3-25 (58%). The rest (2%) is constituted essentially by 2-hydroxymethylfurfural (HMF) and melanoidines. The profile of isomer distribution is practically identical to that shown in Figure 3.

### Example 7:

The procedure of example 6 was repeated exactly, but the resin was replaced by a water soluble poly(*p*-toluenesulfonate) polymer of molecular weight 7 · 10⁴ Dalton in its acid form. The reaction proceeds in this case under homogenous conditions. The catalyst was separated at the end of the caramelization process by using a dialysis membrane with a porosity corresponding to a cut mass of 5000 Dalton. The resulting product is a mahogany-colored caramel that contains fructose (16%), difructose dianhydrides (29%) and higher oligosaccharides of DP 3-20 (52%). The rest (3%) is constituted essentially by 2-hydroxymethylfurfural (HMF) and melanoidines. The profile of isomer distribution is practically identical to that shown in Figure 3.

An identical result was obtained when a water soluble poly(*p*-toluenesulfonate) polymer of molecular weight 10⁶ Dalton was used.

### Example 8:

A suspension of 90% (weight/volume) sucrose (135 g) in water (15 mL) was heated at 90 °C until saturation. The commercial dry ion-exchange resin Lewatit® S2328 was the added as catalyst (13,5 g, 10% by weight relative to the initial sucrose). The heterogeneous mixture was heated at 90 °C in a closed vessel with constant magnetic stirring for 72 hours, after which it was allowed to cool down to room temperature and the catalyst was separated by filtration. The product obtained in this way is a dark mahogany-colored caramel that contains fructose (1%), glucose (23%), difructose dianhydrides (11%), and higher oligosaccharides of DP 2-25 (57%). The rest (8%) is constituted essentially by 2-hydroxymethylfurfural (HMF) and melanoidines.

The mild acid hydrolysis of an aliquot of the obtained caramel or of the fraction containing oligosaccharides of DP higher than 3 led exclusively to fructose, glucose and difructose dianhydrides, which indicates that these oligosaccharides have, in this case, a structure of fructosyl- or glucosyl-difructose dianhydrides. Reversion oligosaccharides are also present in this fraction This is also in agreement with the data of mass spectrometry. The isomeric distribution profile of the difructose dianhydrides arising from hydrolysis is practically identical to that of the difructose dianhydride fraction in the initial caramel and agrees with that shown in Figure 3.

### Example 9:

A 1:1 (weight/weight) mixture of lactose and fructose (135 g total mass) was suspended in water (15 mL; meaning a 90% weight/volume proportion of total sugar) and the resulting suspension was heated at 90 °C until almost total dissolution. The commercial dry ion-exchange resin Lewatit® S2328 was then added as catalyst (13,5 g, 10% by weight relative to the initial total sugar material). The heterogeneous mixture was heated at 90 °C in a closed vessel with constant magnetic stirring during 72 hours, after which it was allowed to cool down to room temperature and the catalyst was separated by filtration. The product obtained in this way is a dark mahogany-colored caramel that contains fructose (1%), difructose dianhydrides (1%), lactose (1%), glucose and galactose (25% altogether) and higher oligosaccharides of DP 2-25 (68%). The rest (4%) is constituted essentially by 2-hydroxymethylfurfural (HMF) and melanoidines.

The mild acid hydrolysis of an aliquot of the obtained caramel or the fraction containing higher oligosaccharides of DP higher than 3 led exclusively to fructose, glucose, galactose and difructose dianhydrides, which indicates that these oligosaccharides have, in this case, a structure of fructosyl-, glucosyl-, galactosyl- and lactosyl-difructose dianhydrides. Reversion oligosaccharides are also present in this fraction. This is also in agreement with the mass spectrometry data. The profile of isomeric distribution of the difructose dianhydrides arising from hydrolysis is practically identical to that of the difructose dianhydride fraction in the initial caramel and agrees with that shown in Figure 3.

### Example 10: In vivo evaluation of the intestinal anti-inflammatory effect of caramels of the invention obtained according to examples 3 and 8 in the experimental model of colitis induced by dextransulfate sodium (DSS) in rats.

The animals that were used in these experiences are Wistar rats, of 200-230 g of weight, provided by the Animal Experimentation Service of the University of Granada. The selected model of experimental inflammation consists in the administration of DSS (5% weight/volume) in the drink water during one week. This model is characterized by generating an inflammatory process in the colon of the rat, with numerous similarities with the intestinal inflammatory disease in humans (Crohn disease), regarding the tissue damage that it generates and the production of mediators involved in the inflammatory response. In order to carry out these studies, different animal groups (n = 10) received the diet supplemented with the appropriate proportion of prebiotic caramel of the examples. This treatment began two weeks before the incorporation of the DSS in the drink water and it was continued for one more week. At this moment the animals were sacrificed and the colonic damage was evaluated. In order to be able to evaluate the efficiency of the treatment with the prebiotic caramels, control groups of colitic animals (n = 10) that received the standard diet containing cellulose instead of the caramel were used. Additionally, a control group (n = 10) that did not receive any dietetic treatment and that was not induced intestinal inflammation was used.

The macroscopic evaluation of the intestinal inflammatory process was made by determination of the colon weight/length relationship (macroscopic damage index, MDI). The followed protocol was, basically, that reported in the publication by D. Camuesco et al. in J. Nutr. 2005, 135, 687-94. For the colon weight/length relationship of the control animals that have not suffered injury the MDI is defined as 0.0, whereas this index reaches an average value of 7.5 in the control group that was treated with DSS and that did not receive prebiotic caramels in their diet. In animals that received caramels of examples 3 and 8, this value decreased to 5.5 and 6.0 respectively, which considering the aggressiveness of the model of inflammatory colitis used represents a very significant capacity for protection/regeneration after the inflammation of the colon.

### Example 11: In vivo evaluation of the effect of caramels of the invention obtained according to examples 3 and 8 in the bacterial flora in rats.

On the animals subjected to treatment with DSS and to which a diet containing the prebiotic caramels prepared according to examples 3 and 8 was provided, as well as on the corresponding control groups, the counts of Lactobacillus and Bifidobacterium was carried out. The population of these bacteria decreased in the animals treated with DSS to 30 and 20% of the values observed in healthy non-treated animals, respectively. In the case of animals fed with the caramels of examples 3 and 8, a very significant recovery of the corresponding bacterium populations was observed, reaching counts close to the initials values.

## Claims

1. Procedure for the preparation of caramels with a high content in prebiotic oligosaccharides of the type difructose dianhydrides and glycosylated difructose dianhydrides from: (a) a food-grade sugar that contains D-fructose and (b) an acid catalyst that acts as caramelization promoter, which involves the heating of a dissolution or a concentrated suspension of the starting sugar material in water in the presence of the catalyst and the subsequent separation of the acid catalyst from the caramel thus obtained at the end of the process by physical methods.

2. Procedure for the preparation of caramels with a high content in prebiotic oligosaccharides of the type difructose dianhydrides and glycosylated difructose dianhydrides according to claim 1, wherein the starting sugar is D-fructose, sucrose or any oligo- or polysaccharide that contains fructose as a constituent, including the glycosylfructoses such as palatinose or leucrose, fructooligosaccharides such as 1-kestose or nystose, fructans and inulin, and these starting sugars are used alone or combined in different proportions, as well as in combination with other food-grade sugars, including glucose, galactose, maltose, lactose or raffinose.

3. Procedure for the preparation of caramels with a high content in prebiotic oligosaccharides of the type difructose dianhydrides and glycosylated difructose dianhydrides according to claim 1 or 2, wherein the acid catalyst is a zeolite, a clay as bentonite or an ion-exchange resin in its acid form, under heterogeneous reaction conditions, or a soluble acid polymer of high molecular weight, under homogeneous reaction conditions.

4. Procedure for the preparation of caramels with a high content in prebiotic oligosaccharides of the type difructose dianhydrides and glycosylated difructose dianhydrides according to any one of claims 1 to 3, wherein the total concentration of the starting sugar material in water ranges between 60-95% (weight/volume), preferably between 70-90%.

5. Procedure for the preparation of caramels with a high content in prebiotic oligosaccharides of the type difructose dianhydrides and glycosylated difructose dianhydrides according to any one of claims 1 to 4, wherein the temperature of heating ranges between 60-110 °C, preferably between 70-90 °C.

6. Procedure for the preparation of caramels with a high content in prebiotic oligosaccharides of the type difructose dianhydrides and glycosylated difructose dianhydrides according to any one of claims 1 to 5, wherein the heating period ranges between 5 minutes and one week, preferably between 15 minutes and 48 hours.

7. Procedure for the preparation of caramels with a high content in prebiotic oligosaccharides of the type difructose dianhydrides and glycosylated difructose dianhydrides according to any one of claims 1 to 6, wherein the proportion in weight of the catalyst, referred to the total starting sugar, ranges between 5-35% by weight.

8. Procedure for the preparation of caramels with a high content in prebiotic oligosaccharides of the type difructose dianhydrides and glycosylated difructose dianhydrides according to any one of claims 1 to 7, wherein the caramelization process is carried out by a continuous method, packing the solid catalyst in a column through which a concentrated dissolution of the sugar material is passed.

9. Procedure for the preparation of caramels with a high content in prebiotic oligosaccharides of the type difructose dianhydrides and glycosylated difructose dianhydrides according to any one of claims 1 to 7, wherein the caramelization process is carried by a discontinuous method, by batches, using an effective agitation during the period of heating.

10. Procedure for the preparation of caramels with a high content in prebiotic oligosaccharides of the type difructose dianhydrides and glycosylated difructose dianhydrides according to any one of claims 1 to 9, wherein the catalyst is a zeolite in its acid form with a Si/Al modulus that ranges between 5-150, preferably between 25-120, in a proportion in weight referred to the total starting sugar material ranging between 25-35%.

11. Procedure for the preparation of caramels with a high content in prebiotic oligosaccharides of the type difructose dianhydrides and glycosylated difructose dianhydrides according to any one of claims 1 to 9, wherein the catalyst is a bentonite in its acid form in a proportion in weight referred to the total starting sugar material ranging between 25-35%.

12. Procedure for the preparation of caramels with a high content in prebiotic oligosaccharides of the type difructose dianhydrides and glycosylated difructose dianhydrides according to any one of claims 1 to 9, wherein the catalyst is an intact ion-exchange resin, in its acid form, in a proportion in weight referred to the total starting sugar material ranging between 10-20%.

13. Procedure for the preparation of caramels with a high content in prebiotic oligosaccharides of the type difructose dianhydrides and glycosylated difructose dianhydrides according to any one of claims 1 to 9, wherein the catalyst is a milled ion-exchange resin, preferably to a size of inferior particle to 80 µm, in its acid form, in a proportion in weight referred to the total starting sugar material ranging between 5-10%.

14. Procedure for the preparation of caramels with a high content in prebiotic oligosaccharides of the type difructose dianhydrides and glycosylated difructose dianhydrides according to claim 13, wherein the resin incorporates acid groups of the sulfonic acid type or acid groups of the carboxylic acid type.

15. Procedure for the preparation of caramels with a high content in prebiotic oligosaccharides of the type difructose dianhydrides and glycosylated difructose dianhydrides according to any one of claims 1 to 7 or 9, wherein the catalyst is a soluble acid polymer of high molecular weight, preferably higher or equal to 10⁴ Dalton, in its acid form, in a proportion in weight referred to the total starting sugar material ranging between 5-10%.

16. Procedure for the preparation of caramels with a high content in prebiotic oligosaccharides of the type difructose dianhydrides and glycosylated difructose dianhydrides according to claim 15, wherein the polymer incorporates acid groups of the sulfonic acid type.

17. Procedure for the preparation of caramels with a high content in prebiotic oligosaccharides of the type difructose dianhydrides and glycosylated difructose dianhydrides according to claim 1, wherein the caramelization process consists in heating a dissolution of fructose at 70-90% (weight/volume) in water at 70-90 °C in the presence of an ion-exchange resin with sulfonic groups in its acid form, using a proportion of catalyst of 5-20% by weight referred to the starting sugar, by a period of 0.5-3 hours, followed by separation of the resin by filtration for further recycling.

18. Procedure for the preparation of caramels with a high content in prebiotic oligosaccharides of the type difructose dianhydrides and glycosylated difructose dianhydrides according to claim 1, wherein the caramelization process consists in heating a dissolution of fructose and lactose, in relative proportions by weight that can vary from 1:5 to 5:1, at a concentration ranging between 85-95% (weight/volume) in water at 80-90 °C, in the presence of an ion-exchange resin with sulfonic groups in its acid form, using a proportion of catalyst of 10-20% by weight referred to the total starting sugar material, for a period of 3-48 hours, followed by separation of the resin by filtration for further recycling.

19. A caramel obtainable by the procedure of claim 1.

20. A caramel according to claim 19 wherein the content of prebiotic oligosaccharides of the type difructose dianhydrides and glycosylated difructose dianhydrides of DP ranging from 3 and 25 is equal or higher than 40%, with a distribution of isomeric difructose dianhydrides close to that corresponding for a thermodynamic equilibrium, in which the major isomer is α-D-fructofuranose β-D-fructopyranose 1.2': 2,1'-dianhydride.

21. A caramel according to claim 20 containing, additionally, other sugars different from fructose, such as for example glucose, galactose, sucrose, maltose, lactose or raffinose, or even reversion oligosaccharides formed by the condensation of these sugars.

22. A caramel according to claim 20 or 21, totally or partially decolorized by treatment with vegetal charcoal or with an appropriate commercial resin for the adsorption of colored products.

23. A caramel according to any one of claims 20 to 22 containing, additionally, at least a component selected between the families of vitamins, flavors, colorants, prebiotics or probiotics.

24. A product intended for the human or animal diet, containing a caramel according to any one of claims 20 to 23 in proportion ranging preferably between 1 and 30% by weight, able to induce a significant increase of Bifidobacteria or Lactobacillus in the intestinal track.

25. A product for use in the prevention or the treatment of pathologies affecting the digestive system, in animals or humans, containing a caramel according to any one of claims 20 to 23 in a proportion ranging preferentially between 1 and 30%, able to induce prebiotics effects.

26. A caramel according to any one of claims 19 to 22, for use in increasing the population of beneficial bacteria, such as Bifidobacteria or Lactobacillus, in the intestinal track of humans or animals.

27. A caramel according to any one of claims 19 to 22, for use in preventing injuries in the digestive system of humans or animals.

## Patentansprüche

1. Verfahren zur Zubereitung von Karamellen mit hohem Gehalt an prebiotischen Oligosacchariden der Art wie Difructosedianhydride und glykosylierte Difructosedianhydride aus: (a) einem Zucker in Lebensmittelqualität enthaltend D-Fructose und (b) einem Säurekatalysator, welcher als Karamellisierungsförderer wirkt, umfassend das Erwärmen einer Lösung oder einer konzentrierten Suspension des anfänglichen Zuckermaterials in Wasser in Anwesenheit von dem Katalysator und das nachfolgende Trennen des Säurekatalysators vom dem derart erhaltenen Karamell am Ende des Prozesses durch physikalische Methoden.

2. Verfahren zur Zubereitung von Karamellen mit hohem Gehalt an prebiotischen Oligosacchariden der Art wie Difructosedianhydride und glykosylierte Difructosedianhydride nach Anspruch 1, wobei der anfängliche Zucker D-Fructose, Saccharose oder ein beliebiges Oligo- oder Polysaccharid enthaltend Fructose als Bestandteil, einschließlich den Glykosylfructosen wie Palatinose oder Leucrose, Fructooligosacchariden wie 1-Kestose oder Nystose, Fructanen und Inulin, ist, und diese anfänglichen Zucker allein oder in verschiedenen Verhältnissen kombiniert, sowie in Kombination mit anderen Zuckern in Lebensmittelqualität, einschließlich Glucose, Galactose, Maltose, Lactose oder Raffinose, verwendet werden.

3. Verfahren zur Zubereitung von Karamellen mit hohem Gehalt an prebiotischen Oligosacchariden der Art wie Difructosedianhydride und glykosylierte Difructosedianhydride nach Anspruch 1 oder 2, wobei der Säurekatalysator ein Zeolith, ein Ton wie Bentonit oder ein Ionenaustauschharz in dessen Säureform, unter heterogenen Reaktionsbedingungen, oder ein lösliches hochmolekulares Säurepolymer, unter homogenen Reaktionsbedingungen, ist.

4. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 3, wobei die Gesamtkonzentration des anfänglichen Zuckermaterials in Wasser zwischen 60-95% (Gewicht/Volumen), vorzugsweise zwischen 70-90%, schwankt.

5. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 4, wobei die Erwärmungstemperatur zwischen 60-110ºC, vorzugsweise zwischen 70-90ºC, schwankt.

6. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 5, wobei die Erwärmungsperiode zwischen 5 Minuten und einer Woche, vorzugsweise zwischen 15 Minuten und 48 Stunden, schwankt.

7. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 6, wobei das Gewichtsverhältnis des Katalysators, in Bezug auf den gesamten anfänglichen Zucker, zwischen 5-35 Gew.-% schwankt.

8. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 7, wobei der Karamellisierungsprozess mittels einer kontinuierlichen Methode durchgeführt wird, in welcher der feste Katalysator in einer Säule, durch welche eine konzentrierte Lösung des Zuckermaterials gefördert wird, gepackt wird.

9. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 7, wobei der Karamellisierungsprozess mittels einer diskontinuierlichen Methode, durch Ladungen, durchgeführt wird, unter Verwendung eines wirksamen Schüttelns während der Erwärmungsperiode.

10. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 9, wobei der Katalysator ein Zeolith in dessen Säureform ist, mit einem Si/Al-Modul, welches zwischen 5-150, vorzugsweise zwischen 25-120 schwankt, in einem Gewichtsverhältnis in Bezug auf das gesamte anfängliche Zuckermaterial, welches zwischen 25-35% schwankt.

11. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 9, wobei der Katalysator ein Bentonit in dessen Säureform ist, in einem Gewichtsverhältnis in Bezug auf das gesamte anfängliche Zuckermaterial, welches zwischen 25-35% schwankt.

12. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 9, wobei der Katalysator ein intaktes Ionenaustauschharz, in dessen Säureform ist, in einem Gewichtsverhältnis in Bezug auf das gesamte anfängliche Zuckermaterial, welches zwischen 10-20% schwankt.

13. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 9, wobei der Katalysator ein gemahlenes Ionenaustauschharz, vorzugsweise gemahlen bis zu einer Teilchengröße kleiner als 80 µm, in dessen Säureform ist, in einem Gewichtsverhältnis in Bezug auf das gesamte anfängliche Zuckermaterial, welches zwischen 5-10% schwankt.

14. Verfahren zur Zubereitung von Karamellen mit hohem Gehalt an prebiotischen Oligosacchariden der Art wie Difructosedianhydride und glykosylierte Difructosedianhydride nach Anspruch 13, wobei das Harz Säuregruppen des Sulfonsäure-Typs oder Säuregruppen des Carbonsäure-Typs enthält.

15. Verfahren zur Zubereitung von Karamellen mit hohem Gehalt an prebiotischen Oligosacchariden der Art wie Difructosedianhydride und glykosylierte Difructosedianhydride nach einem der Ansprüche 1 bis 7 oder 9, wobei der Katalysator ein lösliches hochmolekulares Säurepolymer, vorzugsweise mit einem Molekulargewicht höher als oder gleich 10⁴ Dalton, in dessen Säureform ist, in einem Gewichtsverhältnis in Bezug auf das gesamte anfängliche Zuckermaterial, welches zwischen 5-10% schwankt.

16. Verfahren zur Zubereitung von Karamellen mit hohem Gehalt an prebiotischen Oligosacchariden der Art wie Difructosedianhydride und glykosylierte Difructosedianhydride nach Anspruch 15, wobei das Polymer Säuregruppen des Sulfonsäure-Typs enthält.

17. Verfahren zur Zubereitung von Karamellen mit hohem Gehalt an prebiotischen Oligosacchariden der Art wie Difructosedianhydride und glykosylierte Difructosedianhydride nach Anspruch 1, wobei der Karamellisierungsprozess aus dem Erwärmen einer 70-90% (Gewicht/Volumen) Fructoselösung in Wasser bei 70-90ºC in Anwesenheit von einem Ionenaustauschharz mit Sulfongruppen in dessen Säureform besteht, unter Verwendung eines Katalysatorverhältnis von 5-20 Gew.-% in Bezug auf den anfänglichen Zucker, während einer Periode von 0,5-3 Stunden, gefolgt von der Trennung des Harzes mittels Filtration für eine zusätzliche Rückführung.

18. Verfahren zur Zubereitung von Karamellen mit hohem Gehalt an prebiotischen Oligosacchariden der Art wie Difructosedianhydride und glykosylierte Difructosedianhydride nach Anspruch 1, wobei der Karamellisierungsprozess aus dem Erwärmen einer Fructose- und Lactoselösung, in relativen Gewichtsverhältnissen welche von 1:5 bis 5:1 variieren können, bei einer Konzentration, welche zwischen 85-95% (Gewicht/Volumen) in Wasser bei 80-90ºC schwankt, in Anwesenheit von einem Ionenaustauschharz mit Sulfongruppen in dessen Säureform besteht, unter Verwendung eines Katalysatorverhältnisses von 10-20 Gew.-% in Bezug auf das gesamte anfängliche Zuckermaterial, während einer Periode von 3-48 Stunden, gefolgt von der Trennung des Harzes mittels Filtration für eine zusätzliche Rückführung.

19. Karamell, welcher gemäß dem Verfahren nach Anspruch 1 erhältlich ist.

20. Karamell nach Anspruch 19, wobei der Gehalt an prebiotischen Oligosacchariden der Art wie Difructosedianhydride und glykosylierte Difructosedianhydride mit einem DP welches zwischen 3 und 25 schwankt, gleich oder höher als 40% ist, mit einer Verteilung von isomeren Difructosedianhydriden, welche nahe an derjenigen die einem thermodynamischen Gleichgewicht entspricht, in welcher das Hauptisomer α-D-Fructofuranose β-D-Fructopyranose-Dianhydrid 1,2': 2,1' ist.

21. Karamell nach Anspruch 20, zusätzlich enthaltend andere Zucker die keine Fructose sind, wie zum Beispiel Glucose, Galactose, Saccharose, Maltose, Lactose oder Raffinose, oder sogar Umkehrungsoligosaccharide, welche mittels der Kondensierung dieser Zucker gebildet werden.

22. Karamell nach Anspruch 20 oder 21, welcher völlig oder teilweise mittels Behandlung mit pflanzlicher Kohle oder mit einem geeigneten kommerziellen Harz für die Adsorption von gefärbten Produkten entfärbt ist.

23. Karamell nach einem der Ansprüche 20 bis 22, zusätzlich enthaltend mindestens einen Bestandteil, welcher aus den Familien der Vitamine, Geschmackstoffe, Farbstoffe, Prebiotika oder Probiotika gewählt wird.

24. Produkt für menschliche oder tierische Ernährung, enthaltend einen Karamell nach einem der Ansprüche 20 bis 23, in einem Verhältnis welches vorzugsweise zwischen 1 und 30 Gew.-% schwankt, das in der Lage ist eine bedeutende Erhöhung der Bifidobakterien oder Lactobacillen im Darmtrakt hervorzurufen.

25. Produkt für dessen Verwendung bei der Vorbeugung oder der Behandlung von Pathologien, insbesondere von Pathologien welche das Verdauungssystem, in Tieren oder Menschen, angreifen, enthaltend einen Karamell nach einem der Ansprüche 20 bis 23, in einem Verhältnis, welches bevorzugt zwischen 1 und 30% schwankt, das in der Lage ist prebiotische Effekte hervorzurufen.

26. Karamell nach einem der Ansprüche 19 bis 22, für dessen Verwendung bei der Erhöhung der Bevölkerung von nützlichen Bakterien, wie Bifidobakterien oder Lactobacillen, im Darmtrakt von Menschen oder Tieren.

27. Karamell nach einem der Ansprüche 19 bis 22, für dessen Verwendung bei der Vorbeugung von Schäden im Verdauungssystem von Menschen oder Tieren.

## Revendications

1. Procédé pour la préparation de caramels avec un contenu élevé d'oligosaccharides prébiotiques du type dianhydrides de difructose et dianhydrides de difructose glycosilés à partir de : (a) un sucre de qualité alimentaire qui contient du D-fructose et (b) un catalyseur acide qui agit comme promoteur de caramélisation, ce qui implique le réchauffement d'une dissolution ou une suspension concentrée de la matière de sucre de départ dans l'eau en présence du catalyseur et la séparation postérieur du catalyseur acide du caramel ainsi obtenu à la fin du procédé par des procédés physiques.

2. Procédé pour la préparation de caramels avec un contenu élevé d'oligosaccharides prébiotiques du type dianhydrides de difructose et dianhydrides de difructose glycosilés selon la revendication 1, dans lequel le sucre de départ est du D-fructose, du sucrose ou tout oligo- ou polysaccharide contenant du fructose comme constituant, dont les glycosylfructoses tels que le Palatinose ou le leucrose, les fructooligosaccharides tels que le 1-kestose ou le nystose, les fructanes et l'inuline, et ces sucres de départ sont utilisés seuls ou combinés dans des différentes proportions, ainsi que combinés avec d'autres sucres de qualité alimentaire, dont du glucose, du galactose, du maltose, du lactose ou du raffinose.

3. Procédé pour la préparation de caramels avec un contenu élevé d'oligosaccharides prébiotiques du type dianhydrides de difructose et dianhydrides de difructose glycosilés selon la revendication 1 ou 2, dans lequel le catalyseur acide est une zéolite, une argile comme la bentonite ou une résine échangeuse d'ions sous sa forme acide, dans des conditions de réaction hétérogènes, ou un polymère acide soluble à poids moléculaire élevé, dans des conditions de réaction homogènes.

4. Procédé pour la préparation de caramels avec un contenu élevé d'oligosaccharides prébiotiques du type dianhydrides de difructose et dianhydrides de difructose glycosilés selon l'une quelconque des revendications 1 à 3, dans lequel la concentration totale de la matière de sucre de départ dans l'eau varie entre 60 et 95% (poids/volume), de préférence entre 79 et 90%.

5. Procédé pour la préparation de caramels avec un contenu élevé d'oligosaccharides prébiotiques du type dianhydrides de difructose et dianhydrides de difructose glycosilés selon l'une quelconque des revendications 1 à 4, dans lequel la température de réchauffement varie entre 60 et 110ºC, de préférence entre 70 et 90ºC,

6. Procédé pour la préparation de caramels avec un contenu élevé d'oligosaccharides prébiotiques du type dianhydrides de difructose et dianhydrides de difructose glycosilés selon l'une quelconque des revendications 1 à 5, dans lequel la période de réchauffement varie entre 5 minutes et une semaine, de préférence entre 15 minutes et 48 heures.

7. Procédé pour la préparation de caramels avec un contenu élevé d'oligosaccharides prébiotiques du type dianhydrides de difructose et dianhydrides de difructose glycosilés selon l'une quelconque des revendications 1 à 6, dans lequel la proportion en poids du catalyseur, par rapport au sucre de départ total, varie ente 5 et 35% en poids.

8. Procédé pour la préparation de caramels avec un contenu élevé d'oligosaccharides prébiotiques du type dianhydrides de difructose et dianhydrides de difructose glycosilés selon l'une quelconque des revendications 1 à 7, dans lequel le procédé de caramélisation est mis en oeuvre par un procédé continu, en remplissant le catalyseur solide dans une colonne à travers laquelle passe une dissolution concentrée de la matière de sucre.

9. Procédé pour la préparation de caramels avec un contenu élevé d'oligosaccharides prébiotiques du type dianhydrides de difructose et dianhydrides de difructose glycosilés selon l'une quelconque des revendications 1 à 7, dans lequel le procédé de caramélisation est mis en oeuvre par un procédé discontinu, par lots, utilisant un brassage effectif pendant la période de réchauffement.

10. Procédé pour la préparation de caramels avec un contenu élevé d'oligosaccharides prébiotiques du type dianhydrides de difructose et dianhydrides de difructose glycosilés selon l'une quelconque des revendications 1 à 9, dans lequel le catalyseur est une zéolite sous sa forme acide avec un module Si/Al qui varie entre 5 et 150, de préférence entre 25 et 120, dans une proportion en poids par rapport à la matière de sucre de départ totale variant entre 25 et 35%.

11. Procédé pour la préparation de caramels avec un contenu élevé d'oligosaccharides prébiotiques du type dianhydrides de difructose et dianhydrides de difructose glycosilés selon l'une quelconque des revendications 1 à 9, dans lequel le catalyseur est une bentonite sous sa forme acide dans une proportion en poids par rapport à la matière de sucre de départ totale variant entre 25 et 35%.

12. Procédé pour la préparation de caramels avec un contenu élevé d'oligosaccharides prébiotiques du type dianhydrides de difructose et dianhydrides de difructose glycosilés selon l'une quelconque des revendications 1 à 9, dans lequel le catalyseur est une résine échangeuses d'ions sous sa forme acide dans une proportion en poids par rapport à la matière de sucre de départ totale variant entre 10 et 20%.

13. Procédé pour la préparation de caramels avec un contenu élevé d'oligosaccharides prébiotiques du type dianhydrides de difructose et dianhydrides de difructose glycosilés selon l'une quelconque des revendications 1 à 9, dans lequel le catalyseur est une résine échangeuses d'ions broyée, de préférence à une dimension de particule inférieure à 80 µm, sous sa forme acide, dans une proportion en poids par rapport à la matière de sucre de départ totale variant entre 5 et 10%.

14. Procédé pour la préparation de caramels avec un contenu élevé d'oligosaccharides prébiotiques du type dianhydrides de difructose et dianhydrides de difructose glycosilés selon la revendication 13, dans lequel la résine incorpore des groupes acides du type acide sulfonique ou des groupes acides d type acide carboxylique.

15. Procédé pour la préparation de caramels avec un contenu élevé d'oligosaccharides prébiotiques du type dianhydrides de difructose et dianhydrides de difructose glycosilés selon l'une quelconque des revendications 1 à 7 ou 9, dans lequel le catalyseur est un polymère acide soluble à poids moléculaire élevé, de préférence supérieur ou égal à 10⁴ Dalton, sous sa forme acide, dans une proportion en poids par rapport à la matière de sucre de départ totale variant entre 5 et 10%.

16. Procédé pour la préparation de caramels avec un contenu élevé d'oligosaccharides prébiotiques du type dianhydrides de difructose et dianhydrides de difructose glycosilés selon la revendication 15, dans lequel le polymère incorpore des groupes acides du type acide sulfonique.

17. Procédé pour la préparation de caramels avec un contenu élevé d'oligosaccharides prébiotiques du type dianhydrides de difructose et dianhydrides de difructose glycosilés selon la revendication 1, dans lequel le procédé de caramélisation consiste au réchauffement d'une dissolution de fructose à 70-90% (poids/volume) dans l'eau à 70-90ºC en présence d'une résine échangeuse d'ions avec des groupes sulfoniques sous sa forme acide, en utilisant une proportion de catalyseur de 5 à 20% en poids par rapport le sucre de départ, pendant une période de 0,5 à 3 hures, suivi d'une séparation de la résine par filtration pour un recyclage postérieur.

18. Procédé pour la préparation de caramels avec un contenu élevé d'oligosaccharides prébiotiques du type dianhydrides de difructose et dianhydrides de difructose glycosilés selon la revendication 1, dans lequel le procédé de caramélisation consiste au réchauffement d'une dissolution de fructose et de lactose, dans des proportions relatives en poids pouvant varier de 1:5 à 5:1, à une concentration variant entre 85 et 95% (poids/volume) dans l'eau à 80-90ºC en présence d'une résine échangeuse d'ions avec des groupes sulfoniques sous sa forme acide, en utilisant une proportion de catalyseur de 10 à 20% en poids par rapport à la matière de sucre de départ totale, pendant une période de 3 à 48 heures, suivi d'une séparation de la résine par filtration pour un recyclage postérieur.

19. Un caramel pouvant être obtenu par le procédé de la revendication 1.

20. Un caramel selon la revendication 19, dans lequel le contenu d'oligosaccharides prébiotiques du type dianhydrides de difructose et dianhydrides de difructose glycosilés à GP variant entre 3 et 25 est égal ou supérieur à 40%, avec une distribution de dianhydrides de difructose isomériques proche de celle correspondant à un équilibre thermodynamique, dans lequel l'isomère majeur est du dianhydride d'α-D-fructofuranose, β-D-fructopyranose 1,2':2,1'.

21. Un caramel selon la revendication 20 contenant, en outre, d'autre sucres différents du fructose, tels que du glucose, du galactose, du sucrose, du maltose, du lactose ou du raffinose, ou encore des oligosaccharides de réversion formés par la condensation de ces sucres.

22. Un caramel selon la revendication 20 ou 21, complètement ou partiellement décoloré par un traitement avec du charbon végétal ou avec une résine commerciale appropriée pour l'adsorption de produits colorés.

23. Un caramel selon l'une quelconque des revendications 20 à 22 contenant, en outre, au moins un composant choisi parmi les familles des vitamines, aromes, colorants, prébiotiques ou probiotiques.

24. Un produit destiné à la diète humaine ou animale, contenant un caramel selon l'une quelconque des revendications 20 à 23 dans une proportion variant de préférence entre 1 et 30% en poids, apte à induire une augmentation significative de bifidobactéries ou de lactobacilles dans le tractus intestinal.

25. Un produit destiné à la prévention ou le traitement de pathologies affectant le système digestif, chez les animaux ou les humains, contenant un caramel selon l'une quelconque des revendications 20 à 23 dans une proportion variant de préférence entre 1 et 30% en poids, apte à induire des effets prébiotiques.

26. Un caramel selon l'une quelconque des revendications 19 à 22, destiné à l'augmentation de la population de bactéries bénéfiques, telles que les bifidobactéries ou les lactobacilles, dans le tractus intestinal des humains ou des animaux.

27. Un caramel selon l'une quelconque des revendications 19 à 22, destiné à la prévention de lésions dans le système digestif des humains ou des animaux.
